**Europäisches Patentamt**

**European Patent Office**

**Office européen des brevets**

(19)

(11) Veröffentlichungsnummer : **0 000 536**
**B1**

(12)

# EUROPÄISCHE PATENTSCHRIFT

(45) Veröffentlichungstag der Patentschrift :
02.12.81

(21) Anmeldenummer : **78100423.9**

(22) Anmeldetag : **18.07.78**

(51) Int. Cl.³ : **C 07 D303/04, C 07 D301/32**

(54) **Verfahren zur Auftrennung vinyloxiranhaltiger Lösungen.**

(30) Priorität : **29.07.77 DE 2734241**

(43) Veröffentlichungstag der Anmeldung :
**07.02.79 (Patentblatt 79/03)**

(45) Bekanntmachung des Hinweises auf die Patenterteilung : **02.12.81 Patentblatt 81/48**

(84) Benannte Vertragsstaaten :
**DE**

(56) Entgegenhaltungen :
FR - A - 2 309 550
GB - A - 846 534

(73) Patentinhaber : **BAYER AG**
**Zentralbereich Patente, Marken und Lizenzen**
**D-5090 Leverkusen 1, Bayerwerk (DE)**

(72) Erfinder : **Seifert, Hermann, Dr.**
**Ruwergasse 4**
**D-5000 Koeln 80 (DE)**
Erfinder : **Waldmann, Helmut, Dr.**
**Carl-Rumpff-Strasse 59**
**D-5090 Leverkusen 1 (DE)**
Erfinder : **Schwerdtel, Wulf, Dr.**
**Hegelstrasse 9**
**D-5090 Leverkusen 1 (DE)**
Erfinder : **Swodenk, Wolfgang, Dr.**
**Auf dem Broich 5**
**D-5068 Odenthal (DE)**

## Verfahren zur Auftrennung vinyloxiranhaltiger Lösungen

Die vorliegende Erfindung betrifft ein Verfahren zur Auftrennung von Gemischen, die im wesentlichen aus Vinyloxiran (dem Monoepoxid des Butadiens), einer Carbonsäure, Butadien und Benzol bestehen.

Bei der Herstellung von Vinyloxiran nach vorbekannten Verfahren fällt im allgemeinen als Reaktionsprodukt ein Gemisch an, das im wesentlichen Vinyloxiran, eine niedermolekulare Carbonsäure und Butadien enthält (vgl. Pudovik u. Ivanov, Journal of Gen. Chem. of USSR, Vol 26, 3087 (1956) in Engl. translation, und F. C. Frostick et al., Journal of Am. Chem. Soc., 81, 3354 (1959), sowie die in der Japanischen Patentschrift J 7-4046-284 und in der Deutschen Patentschrift 1 216 306 beschriebenen Verfahren). Sehr häufig liegt ein derartiges Gemisch als Lösung vor in einem organischen Lösungsmittel, das einen Siedepunkt aufweist, der zwischen dem von Vinyloxiran und der Carbonsäure liegt.

Alle Verfahren zur Herstellung von Vinyloxiran, bei denen im Reaktionsgemisch neben Vinyloxiran eine Carbonsäure vorliegt, beruhen darauf, daß Butadien mit einer Percarbonsäure, z.B. Peressigsäure oder Perameisensäure, als selektivem Oxidationsmittel für Butadien, gegebenenfalls in einem hydrophoben organischen Lösungsmittel oxidiert wird. Die zusammen mit dem Epoxid anfallende Carbonsäure muß aber so rasch wie möglich entfernt werden. Dazu heißt es beispielsweise bei D. Swern, Organic Peroxides, Whiley Interscience 1971, Vol. II, S. 433. « Aufarbeitung des Produktgemisches kommt bei im industriellen Maßstab durchgeführten Epoxidationen besondere Bedeutung zu. Die vollständige Entfernung von organischen und anorganischen Säuren aus dem Epoxid ist notwendig, um eine nachfolgende Spaltung oder Polymerisation des Epoxids zu verhindern... »

Insbesondere ist es erforderlich zu verhindern, daß das Vinyloxiran mit der aliphatischen Carbonsäure unter Ringspaltung zum entsprechenden Buten-(1)-diol-(3,4)-monoester weiterreagiert. Diese Reaktion zwischen Vinyloxiran und aliphatischen Carbonsäuren kann bereits während der Reaktion aber auch während der Auftrennung der Reaktionsgemische stattfinden. Dies hat zur Folge, daß bei diesen bisher bekannten Vinyloxiran-Herstellungsverfahren die Ausbeute an gereinigtem Vinyloxiran merklich verringert ist. Bei dem Angriff der Carbonsäure auf das Vinyloxiran handelt es sich somit um eine äußerst unerwünschte, ausbeutemindernde Reaktion. Infolgedessen sind auch eine Reihe von Verfahren bekannt geworden, bei denen Maßnahmen angewendet werden, um diese Reaktion bei der Aufarbeitung vinyloxiranhaltiger Reaktionsgemische zur Auftrennung in die einzelnen Bestandteile nach Möglichkeit zu unterbinden.

In der Britischen Patentschrift 852 097 wird, um die Trennung von Vinyloxiran und der bei der Epoxidierung von Butadien mit der Percarbonsäure zwangsläufig entstehenden Carbonsäure unter schonenden Bedingungen zu erreichen, vorgeschlagen, die wasserlösliche Carbonsäure, wie beispielsweise Essigsäure, aus Gemischen dieser Säure und Vinyloxiran dadurch zu entfernen, daß man zunächst eine Lösung von Vinyloxiran und der Carbonsäure in einem organischen, in Wasser nicht löslichen Lösungsmittel, wie beispielsweise chlorierten Kohlenwasserstoffen, herstellt und die Lösung danach mit Wasser oder einer wäßrigen Lösung eines inerten Alkalimetallsalzes bei Temperaturen unter 15 °C extrahiert. Dieses Extraktionsverfahren ist aufwendig, da es nicht nur ein zusätzliches Lösungsmittel erfordert, sondern auch eine nachfolgende Trennung des Wassers von der Carbonsäure notwendig macht, wenn die Carbonsäure wiedergewonnen werden soll. Die Gewinnung von Carbonsäuren aus wäßrigen Lösungen durch Destillation ist, wegen der Bildung azeotroper Gemische, sehr aufwendig.

Der Umfang der für ein derartiges Extraktionsverfahren zur Gewinnung von Vinyloxiran technisch notwendigen Maßnahmen ist der US-Patentschrift 3 019 234 zu entnehmen. Die nach Zusatz des in Wasser weitgehend unlöslichen Lösungsmittels resultierende, Vinyloxiran und die Carbonsäure enthaltende Lösung muß unterhalb 25 °C mit Wasser oder einer wäßrigen Lösung extrahiert werden. Der Extraktionsaufwand ist erheblich. Aus Beispiel 1 der obengenannten Patentschrift geht hervor, daß 10 theoretische Extraktionsstufen erforderlich sind. Zudem muß bei 0 °C gearbeitet werden. Die durch dieses Verfahren zur Gewinnung von Vinyloxiran vorgeschlagene Problemlösung zur verlustfreien Trennung von Vinyloxiran und Carbonsäure ist nicht zufriedenstellend, da sich wesentliche Mengen an Vinyloxiran nach erfolgter Extraktion in der wäßrigen Phase befinden.

In der Französichen Patentschrift 1 468 814 wird vorgeschlagen, das nach Durchführung der Epoxidierung des Butadiens mit Percarbonsäuren anfallende, freie Carbonsäure — in diesem Fall Essigsäure — enthaltende Reaktionsgemisch, das daneben noch einen Essigsäureester als Lösungsmittel, Vinyloxiran und Butadien enthält, mit starken Basen, wie Alkali — oder Erdalkalihydroxiden, zu behandeln, um die freie Carbonsäure zu neutralisieren, die somit der Reaktion mit Vinyloxiran entzogen wird. Nach dem Neutralisationsschritt wird die wäßrige, Acetat enthaltende Phase abgetrennt. Dieses Verfahren liefert somit in jedem Fall ein salzhaltiges Abwasser, was besondere ökologische Probleme mit sich bringt.

In einem weiteren Verfahren, das in der Britischen Patentschrift 846 534 beschrieben ist, werden, um die verlustfreie Trennung von Vinyloxiran und der Carbonsäure zu erreichen, durch das aus Carbonsäure und Vinyloxiran bestehende Gemisch die Dämpfe eines Lösungsmittels geleitet. Durch diese Maßnahme soll eine wesentliche Verbesserung der Ausbeute an Vinyloxiran durch Vermeidung der ausbeutemindernden Reaktion von Vinyloxiran mit der Carbonsäure ebenso erreicht werden, wie eine effektivere Trennung der beiden Produkte. Als für diesen Zweck geeignete Lösungsmittel werden chlorierte Kohlenwasserstoffe, wie Äthylenchlorid, Tetrachlorkohlenstoff, Trichloräthylen u.a. vorgeschlagen. Aus den in den Beispielen der obengenannten Patentschrift gemachten Angaben können die erreichten Selektivitäten an Vinyloxiran, bezogen auf Peressigsäure oder Butadien weder ersehen noch errechnet werden. Ebenso ist aus dieser Patentschrift nicht zu entnehmen, welche Verbesserungen hinsichtlich der Verringerung von Verlusten an Vinyloxiran durch die Anwendung des chlorierten Kohlenwasserstoffs erzielt werden können. Auf jeden Fall bedeutet die hier vorgeschlagene Maßnahme zusätzliche aufwendige Destillationen des als Hilfsmittel verwendeten chlorierten Kohlenwasserstoffs.

Bei der technischen Anwendung dieses Verfahrens ergeben sich außerdem Nachteile, die in den durch die Verwendung halogenhaltiger Lösungsmittel bedingten Korrosionsproblemen zu sehen sind.

Zusammenfassend kann also aus der bisher bekannt gewordenen Literatur über Verfahren zur Auftrennung von vinyloxiranhaltigen Gemischen, entnommen werden, daß alle diese Verfahren für eine wirtschaftliche und in technischem Maßstab durchzuführende Gewinnung von Vinyloxiran nicht geeignet sind.

Demgegenüber wurde nun ein Verfahren zur Auftrennung einer im wesentlichen Vinyloxiran, Butadien, eine 1-5 Kohlenstoffatome aufweisende Carbonsäure und Benzol enthaltenden Lösung durch Destillation gefunden, das dadurch gekennzeichnet ist, daß man die Lösung einer ersten Destillationskolonne zuführt und bei einem Druck von 0,5 bis 2,5 bar Butadien, Vinyloxiran und soviel des in der Lösung enthaltenen Benzols über Kopf abdestilliert, daß im Kopfprodukt dieser ersten Destillation 20 bis 70 Gew.% Benzol enthalten sind, wobei die Carbonsäure und das restliche Benzol als Sumpfprodukt erhalten werden, und daß man das Kopfprodukt der ersten Destillation einer zweiten Destillationskolonne zuführt, in der Butadien und gegebenenfalls kleine Anteile von tiefer als Vinyloxiran siedenden Komponenten über Kopf abdestilliert werden und ein im wesentlichen aus Vinyloxiran und Benzol bestehendes Sumpfprodukt erhalten wird, welches zum Teil als Rücklauf in die erste Destillationskolonne zurückgeführt wird.

Als 1 bis 5 Kohlenstoffatome enthaltende Carbonsäure seien Ameisensäure, Essigsäure, Propionsäure, Isobuttersäure, n-Buttersäure, n-Valeriansäure, Trimethylessig- und Dimethylpropionsäure genannt. Es kommen auch Carbonsäuren und Frage, die substituiert sind, beispielsweise durch Fluor oder Chlor wie Trifluoressigsäure, Monofluoressigsäure, Monochloressigsäure, 1-Chlorpropionsäure, 2-Chlorpropionsäure, 2-Flu-

orpropionsäure, 1-Fluorpropionsäure.

Bevorzugt verwendet man für das erfindungsgemäße Verfahren vinyloxiranhaltige Gemische, die Essigsäure, Propionsäure n-Buttersäure oder Isobuttersäure enthalten. Ganz besonders geeignet ist das erfindungsgemäße Verfahren zur Auftrennung von Essigsäure oder Propionsäure enthaltenden Gemischen.

Die Konzentration an Vinyloxiran und an Carbonsäure in der benzolischen Ausgangslösung kann in weiten Grenzen variieren. Im allgemeinen beträgt die Konzentration an Vinyloxiran 1 bis 50, vorzugsweise 3 bis 30 Gew.%. Ganz besonders geeignet sind Lösungen mit einem Gehalt an Vinyloxiran von 5 bis 20 Gew.%.

Der Gehalt an Carbonsäure beträgt im allgemeinen 3 bis 50, vorzugsweise 5 bis 40 Gew.%. Der Gehalt an Butadien kann bis zur Löslichkeitsgrenze von Butadien in dem jeweils aufzutrennenden Gemisch betragen. Daneben können auch kleine Mengen an anderen Verbindungen vorhanden sein. Besonders vorteilhaft ist es, das erfindungsgemäße Verfahren auf Gemische anzuwenden, die bei der Herstellung von Vinyloxiran durch Umsetzung von Butadien mit einer Percarbonsäure in benzolischer Lösung anfallen. In solchen Mischungen sind im allgemeinen kleine Mengen Acetaldehyd, Methylformiat, Propionaldehyd, Methacrolein, Crotonaldehyd, Buten-(1)-diol-(3,4)-mono- und -diester sowie Buten-(1)-diol-(3,4) selbst vorhanden. Ebenso können solche Mischungen geringe Mengen an freiem Wasserstoffperoxid enthalten.

Der Anteil des Benzols in dem nach dem erfindungsgemäßen Verfahren aufzutrennenden Gemisch beträgt im allgemeinen 20 bis 80 Gew.%. In besonderen Fällen kann dieser Konzentrationsbereich auch über- oder unterschritten werden. Besonders eignen sich für die erfindungsgemäße Auftrennung Gemische mit einem Gehalt von 30 bis 70 Gew.% Benzol.

Die nach dem erfindungsgemäßen Verfahren zur Auftrennung gelangenden Gemische können geringe Mengen Wasser enthalten. Wassergehalte bis 5 Gew.% sind für das Verfahren im allgemeinen ohne Bedeutung. Vorteilhafterweise setzt man jedoch Mischungen ein, die weniger als 3 Gew.% Wasser enthalten. Ganz besonders geeignet ist das erfindungsgemäße Verfahren zur auftrennung von Lösungen, deren Gehalt an Wasser unter 1,0 Gew.% liegt, beispielsweise Lösungen mit einem Wassergehalt von 0,05-1,0 Gew.%.

Bei der üblichen Durchführung des erfindungsgemäßen Verfahrens wird in die erste Destillationskolonne ein Gemisch der beschriebenen Zusammensetzung eindosiert und bei einem Druck von 0,5 bis 2,5, bevorzugt 1,0 bis 2,0 bar, ein Butadien, Vinyloxiran und einen Teil des Benzols enthaltendes Kopfprodukt abdestilliert. Die Menge Benzol, die über Kopf mit abdestilliert wird, beträgt im allgemeinen 20 bis 70, bevorzugt 25 bis 70 Gew.% des Kopfproduktes. Besonders bevorzugt enthält das Destillat 30 bis 50 Gew.% Benzol. Bei der Destillation beträgt die Kopftemperatur der Destillationskolonne im allgemeinen 35 bis 80 °C. Ein besonderer Vorteil des erfindungsgemäßen Verfahrens besteht darin, daß am Kopf der Kolonne mit Wasser gekühlt werden kann, wobei eine Kühlwassertemperatur von 20 bis 35 °C ausreichend ist. Das bedeutet, daß ein Kühlwasser verwendet werden kann, das als Rückstrom aus den Kühltürmen technischer Anlagen in großen Mengen zur Verfügung steht.

Bei der erfindungsgemäßen Destillation wird vermieden, das Kopfprodukt der ersten Destillationskolonne ganz oder teilweise als Rücklauf in die erste Kolonne zurückzuleiten. Vielmehr wird das Kopfprodukt der ersten Kolonne, gegebenenfalls nach Kondensation, in die zweite Destillationskolonne geleitet. In der zweiten Destillationseinheit wird Butadien, gegebenenfalls zusammen mit Verbindungen, die einen tieferen Siedepunkt als Vinyloxiran haben, z.B. die Butene, abdestilliert und als Sumpfprodukt Vinyloxiran und der in der ersten Kolonne überdestillierte Teil des Benzols erhalten. Einen Teil des Sumpfproduktes der zweiten Destillationseinheit gibt man in der erforderlichen Menge als Rücklauf in die erste Destillationseinheit zurück. Der Restanteil des Sumpfproduktes der zweiten Destillationskolonne wird entfernt. Es handelt sich dabei um eine im wesentlichen Vinyloxiran enthaltende benzolische Lösung, die in üblicher Weise, z.B. durch eine Feinfraktionierung, in reines Vinyloxiran einerseits und Benzol andererseits aufgetrennt werden kann. Das Rücklaufverhältnis in der ersten Destillationskolonne beträgt beim erfindungsgemäßen Verfahren im allgemeinen 3 bis 0,1, häufig 2,4 bis 0,3. Vorzugsweise beträgt es 2,0 bis 0,4. Besonders vorteilhaft ist ein Rücklaufverhältnis von 1,5 bis 0,5.

Der Druck in der zweiten Destillationskolonne ist in weiten Grenzen variierbar und es kann bei Normaldruck, erhöhten Drücken oder bei vermindertem Druck destilliert werden. Vorzugsweise wird die zweite Destillationskolonne bei erhöhtem Druck betrieben. Besonders vorteilhaft ist es, die Kolonne bei einem so hohen Druck zu betreiben, daß am Kopf der Kolonne ein wesentlicher Teil des dampfförmigen Kopfproduktes mit Wasser kondensiert werden kann. Es ist aber auch möglich, das gesamte Kopfprodukt mit Wasser oder Sole zu kondensieren. Im allgemeinen wird die zweite Destillationskolonne bei Drücken von 1,1 bis 40 bar betrieben. Ein bevorzugter Druckbereich sind Drücke von 1,5 bis 15 bar. Ganz besonders vorteilhaft wählt man den Druck in der zweiten Destillationskolonne so, daß nach vollständiger Kondensation des butadienhaltigen Kopfproduktes dieser Kolonne dieses bei Temperatur über 0 °C als flüssige Phase erhalten werden kann.

Die Kondensation in der zweiten Destillationseinheit wird in üblicher Weise durchgeführt. Man kann beispielsweise das am Kopf anfallende Butadien gegebenenfalls im Gemisch mit Butenen kondensieren und das flüssige Kopfprodukt als Rücklauf auf die Kolonne zurückgeben und in flüssiger Form aus der Kolonne austragen. Man kann aber auch einen Dephlegmator als Kondensationseinheit verwenden, wobei Butadien gasförmig die Destillationskolonne verläßt. Das gasförmige Butadien kann beispielsweise durch eine Wäsche mit einem geeigneten Lösungsmittel von gegebenenfalls im ausgasenden Butadien vorhandenen Vinyloxiran befreit werden. Das so gewaschene gasförmige Butadien kann man aber auch, beispielsweise durch Kompression, verflüssigen und ganz oder teilweise als Rücklauf in die zweite Kolonne zurückführen.

Ein zweckmäßiges Rücklaufverhältnis in dieser zweiten Destillationskolonne kann leicht ermittelt werden. Es kann grundsätzlich in weiten Grenzen variiert werden. Es kann Werte von beispielsweise 0,1 bis 10 betragen. Ein gegebenenfalls in dieser Größenordnung angewendetes Rücklaufverhältnis bedeutet keinen besonderen technischen Aufwand für das Verfahren, da am Kopf dieser zweiten Destillationskolonne im wesentlichen nur Butadien, gegebenenfalls im Gemisch mit Butenen vorliegt. Die Gesamtmenge dieses Butadien/Buten-Gemisches ist so klein, daß auch ein hohes Rücklaufverhältnis keinen besonderen Aufwand bedeutet.

Als Destillationskolonnen kommen für die erste und die zweite Destillationsstufe alle üblichen Vorrichtungen in Frage, beispielsweise Füllkörperkolonnen oder Bodenkolonnen. Als Füllkörper und Böden sind alle üblichen Formen geeignet. Als Verdampfer kann man ebenfalls die bekannten Vorrichtungen wie Umlaufverdampfer, Dünnschichtverdampfer oder Fallstromverdampfer verwenden.

Man kann das vinyloxiran- und carbonsäurehaltige Gemisch flüssig oder gasförmig in die erste Destillationseinheit leiten. Man kann aber auch spezielle Durchführungsformen der Produkteinspeisung anwenden. So kann man beispielsweise das flüssige Gemisch über eine Verdampfereinheit, beispielsweise einen Dünnschichtverdampfer, leiten und das Gas und Flüssigkeit dieser Verdampfungsstufe getrennt in die Kolonne eingeben. Dies kann insofern vorteilhaft sein, als eine besonders rasche Trennung von Vinyloxiran und Carbonsäure erreichbar ist.

In vielen Fällen kann es von Vorteil sein, das Vinyloxiran- und carbonsäurehaltige Gemisch vor Eintritt in die erste Destillationseinheit mit einem Stabilisator oder einer Lösung eines Stabilisators zu versetzen. Man kann aber auch ohne Anwendung eines Stabilisators das Vinyloxiran enthaltende Gemisch der erfindungsgemäßen, destillativen Auftrennung unterwerfen. Als Stabilisatoren oder Inhibitoren sind in diesem Zusammenhang Verbindungen zu verstehen, die in der Lage sind, die Polymerisationsneigung des Vinyloxirans und des Butadiens moderierend zu beeinflussen. Für diesen Zweck kommen insbesondere alle die Verbindungen in Frage, die aufgrund ihrer chemischen Struktur Sauerstoff oder Spuren peroxidischer Verbindungen binden oder zerstören können. Diese Verbindungen können auch Stickstoff und/oder Schwefel enthalten. Beispielsweise seien erwähnt Hydrochinon, 4-tert.-Butylbrenzcatechin, 2,6-Di-tert.-Butyl-4-methylphenol, Alkyl-anthrahydrochinone, Phenothiazin und dessen Derivate, N,N-Dimethylanilin, Tetramethyl-hydrochinon, N-Nitroso-diphenylamin, Pyrogallol, 2-Methyl-benzothiazol, 6-Methoxy-2-amino-benzothiazol, 2,3-Dihydroxychinoxalin und die Additionsverbindung aus Diisobutylen und Stickstoffoxid. Selbstverständlich können auch Gemische dieser Verbindungen eingesetzt werden. Gelangt der Stabilisator in Lösung zur Anwendung, so ist es vorteilhaft, als Lösungsmittel die Carbonsäure oder Benzol zu benutzen.

Die Mengen an Stabilisator können in weiten Grenzen schwanken. Im allgemeinen ist es ausreichend, bezogen auf die Menge, die in die erste Destillationseinheit gelangt, 0,05 bis 0,5 Gew.% an Stabilisator zur Anwendung zu bringen. Der Stabilisator bzw. dessen Lösung kann, wie schon erwähnt, dem aufzutrennenden, im wesentlichen Vinyloxiran, Butadien, die Carbonsäure und Benzol enthaltendem Gemisch vor Eintritt in die erste Destillationseinheit zugesetzt werden. Diese Maßnahme liefert im allgemeinen befriedigende Ergebnisse. Vorteilhafter ist es, den Stabilisator dem Rücklauf auf die erste Destillationskolonne zuzufügen. Besonders vorteilhaft ist es jedoch, den Stabilisator dem Einlauf in die zweite Destillationskolonne zuzufügen, womit er in die Lage versetzt wird, das erfindungsgemäße Verfahren wie folgt zu durchlaufen : Nach Passieren des Abtreiberteils der zweiten Destillationskolonne gelangt der Stabilisator, da höhersiedend als das Butadien, in den Sumpf dieser Kolonne und wird von dort teilweise mit dem Rücklauf für die erste Destillationseinheit des Verfahrens auf diese erste Kolonne gegeben, die er von oben nach unten durchläuft. Letzlich wird der Stabilisator bzw. dessen Reaktionsprodukte mit dem Sumpfprodukt der ersten Kolonne, das die Carbonsäure und Benzol enthält, aus dem Verfahren ausgetragen.

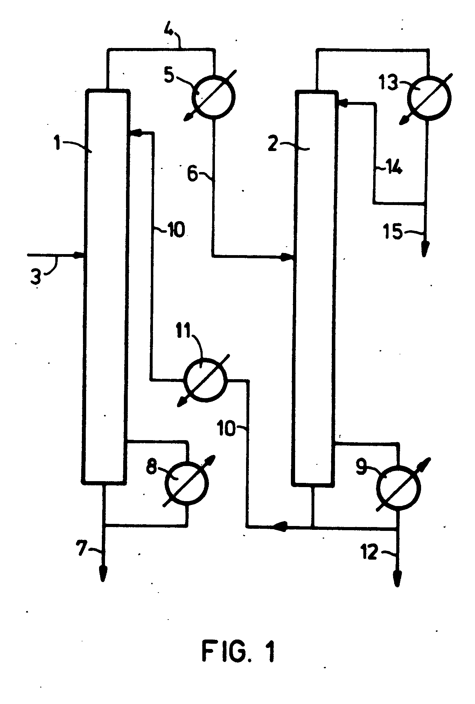

In einer besonderen Durchführungsform des erfindungsgemäßen Verfahrens verwendet man, wie aus Fig. 1 ersichtlich, eine aus 2 Kolonnen (1) und (2) bestehende Destillationsstraße. In die Kolonne (1) gibt man über (3) eine Lösung von 8 bis 14 Gew.% Vinyloxiran, 10 bis 15 Gew.% Butadien, 18 bis 28 Gew.% Propionsäure in Benzol ein, die zuvor mit 0,1 Gew.% 4-tert.-Butylbrenzcatechin versetzt worden ist. Bei einem Druck von 1,0 bis 1,5 bar, einer Sumpftemperatur von 80 bis 110 °C und einer Kopftemperatur von 40 bis 75 °C destilliert man das gesamte Butadien und Vinyloxiran und soviel an Benzol über Kopf, daß im Destillat der Kolonne (1) 40 bis 45 Gew.% Benzol enthalten sind. Das bei (4) anfallende Destillat der Kolonne (1) wird mit Kühlwasser von 24 °C im Kühler (5) kondensiert. Kondensat und gegebenenfalls nichtkondensierte Anteile werden über (6) in die Destillationskolonne (2) geleitet. Aus dem Sumpf der Kolonne (1) wird über (7) die gesamte Carbonsäuremenge und der Rest des Benzols herausgezogen. Der Sumpf der Kolonne (1) wird mittels Verdampfer (8) beheizt.

Die zweite Destillationskolonne (2) wird bei einem Druck von 2 bis 3 bar betrieben. Als Sumpf dieser zweiten mit dem Verdampfer (9) beheizten Kolonne fällt ein butadienfreies Gemisch von ca. 45 bis 55 Gew.% Benzol und ca. 45 bis 55 Gew.% Vinyloxiran an, das über (10) und den Kühler (11) zum Teil als Rücklauf auf die Kolonne (1) geleitet wird. Das Rücklaufverhältnis in der Kolonne (1) beträgt 0,5 bis 1,5. Der andere Teil des butadienfreien Sumpfes der Kolonne (2) wird über (12) entnommen und stellt die gewünschte carbonsäurefreie Lösung von Vinyloxiran in Benzol dar. Am Kopf der Kolonne (2) fällt nach Kühlung mit dem Kühler (13) das Butadien flüssig an, das teilweise über (14) als Rücklauf auf die Kolonne (2) gegeben wird. Das restliche Butadien wird über (15) entnommen.

Der besondere Vorteil des erfindungsgemäßen Verfahrens besteht darin, daß die Bildung von Umsetzungsprodukten von Vinyloxiran mit der Carbonsäure so stark vermindert wird, daß diese Nebenproduktbildung mit den gebräuchlichen analytischen Methoden nicht mehr nachgewiesen werden kann. Ein weiterer Vorteil des erfindungsgemäßen Verfahrens besteht darin, daß die Kondensation in der ersten Kolonne mit Wasser als Kühlmittel durchgeführt werden kann, wobei eine Wassertemperatur von 20 bis 30 °C ausreichend ist. Ein besonderer wirtschaftlicher Vorteil des erfindungsgemäßen Verfahrens ist die verlustfreie Abtrennung des Vinyloxirans von einer niedermolekularen aliphatischen Carbonsäure durch Destillation mit einem überraschend kleinen Rücklaufverhältnis. Überraschenderweise hat es sich dabei als vorteilhaft erwiesen, einen Teil des Benzols in der ersten Destillationskolonne über Kopf abzudestillieren und das butadienfreie Sumpfprodukt der zweiten Destillationskolonne als Rücklauf auf die erste Kolonne zurückzuführen. Durch die erfindungsgemäße Maßnahme, einen Teil des Benzols mit dem Vinyloxiran in der ersten Kolonne mit über Kopf zu nehmen, kann man das Rücklaufverhältnis auf Werte zwischen 1,5 und 0,5 reduzieren. Dies bedeutet eine wesentliche Verringerung des Verdampfungs- und Kondensationsaufwandes und eine entsprechende Reduzierung (kleinere Dimensionen) der ersten Destillationskolonne.

Beispiel 1 (s. auch Fig. 1)

Der Destillationskolonne (1) werden pro Stunde 2 720 g eines Reaktionsgemisches aus der Umsetzung von überschüssigem Butadien mit einer benzolischen Lösung von Perpropionsäure zu Herstellung von Vinyloxiran über (3) zugeführt. Das in die Kolonne (1) eingespeiste Gemisch enthält 12,0 Gew.% Vinyloxiran, 21,7 Gew.% Propionsäure, 52,25 Gew.% Benzol und 13,8 Gew.% Butadien sowie geringe Mengen an Wasser, Buten-(1) diol-(3,4)-monopropionat und tiefsiedenden Nebenprodukten. Diesem Gemisch werden vor Eintritt in die Kolonne (1) 0,5 g/h N-Nitrosodiphenylamin, gelöst in 10 ml Benzol, zugesetzt. Die Kolonne (1) besitzt eine Länge von 3,6 m, wobei 1,2 m auf den Verstärkerteil entfallen. Die Füllung der Kolonne besteht aus 4 × 4 mm Glass-Raschigringen. Der Durchmesser der Kolonne beträgt 5 cm. Die Kolonne ist mit einer Kondensationseinrichtung (5) für das Kopfprodukt sowie einer Verdampfereinheit (8) versehen. Der Druck in der Kolonne beträgt 1 bar. Bei einer Sumpftemperatur von 92-94 °C und einer Temperatur am Kopf der Kolonne von 63-66 °C werden in der Stunde nach Kondensation 2.120 g eines Gemisches als Destillat erhalten, das neben 39,1 Gew.% Vinyloxiran und 17,8 Gew.% Butadien noch 42,2 Gew.% Benzol enthält. Der Wassergehalt in diesem Destillat liegt unter 1 Gew.%.

Aus dem Sumpf der Kolonne (1) werden in der Stunde 1.662 g eines Gemisches abgezogen, das neben Benzol die gesamte Propionsäure enthält. Der Gehalt an Butendiolmonopropionat, dem Reaktionsprodukt von Vinyloxiran mit Propionsäure, und Butendiol, dem Reaktionsprodukt von Wasser mit Vinyloxiran, beträgt lediglich 0,15 Gew.% bzw. 0,04 Gew.%.

Das Destillat der Kolonne (1) (2.120 g/h) wird in die zweite Kolonne eingegeben.

Die Kolonne (2) hat eine Länge von 2,6 m, einen Durchmesser von 2,5 cm und ist mit 4 × 4 mm Glasringen gefüllt. Der Zulauf in die Kolonne liegt in der Mitte. Die Kolonne wird bei einem Druck von 1,7 bar betrieben. Die Sumpftemperatur beträgt 78 bis 80 °C ; die Temperatur am Kopf 3 bis 5 °C. Das Rücklaufverhältnis liegt bei 0,7. Als Kopfprodukt fällt nach Kondensation und Abscheidung geringer Mengen Wassers Butadien (373 g/h) an, das eine Reinheit von über 98,5 Gew.% besitzt.

Das Sumpfprodukt, das in einer Menge von 1 742 g/h abgezogen wird, enthält 47,6 Gew.% Vinyloxiran und 51,3 Gew.% Benzol. Von diesem Produktstrom, der frei von Butadien ist, werden pro Stunde 1 060 g als Rücklauf auf die erste Kolonne gegeben, während der Rest in einer nachfolgenden Destillationsstufe in einfacher Weise in reines Vinyloxiran und Benzol aufgetrennt wird. Das Verhältnis von Rücklauf zur Kopfentnahme in der ersten Kolonne beträgt somit 0,5.

Die während der Abtrennung der Propionsäure feststellbaren Verluste an Vinyloxiran betragen lediglich 0,5 % von der mit dem Reaktionsgemisch in die erste Kolonne eingesetzten Menge an Vinyloxiran.

## Ansprüche

1. Verfahren zur Auftrennung einer im wesentlichen Vinyloxiran, Butadien, eine 1-5 Kohlenstoffatome aufweisende Carbonsäure und Benzol enthaltenden Lösung durch Destillation, dadurch gekennzeichnet, daß man die Lösung einer ersten Destillationskolonne zuführt und bei einem Druck von 0,5 bis 2,5 bar Butadien, Vinyloxiran und soviel des in der Lösung enthaltenen Benzols über Kopf abdestilliert, daß im Kopfprodukt dieser ersten Destillation 20 bis 70 Gew.% Benzol enthalten sind, wobei die Carbonsäure und das restliche Benzol als Sumpfprodukt erhalten werden, und daß man das Kopfprodukt der ersten Destillation einer zweiten Destillationskolonne zuführt, in der Butadien und gegebenenfalls kleine Anteile von tiefer als Vinyloxiran siedenden Komponenten über Kopf abdestilliert werden und ein im wesentlichen aus Vinyloxiran und Benzol bestehendes Sumpfprodukt erhalten wird, welches zum Teil als Rücklauf in die erste Destillationskolonne zurückgeführt wird.

2. Verfahren nach Anspruch 1, dadurch gekennzeichnet, daß man in der ersten Destillationskolonne ein Kopfprodukt entnimmt, das 25 bis 70 Gew.% Vinyloxiran enthält.

3. Verfahren nach Anspruch 1 und 2, dadurch gekennzeichnet, daß man die erste Destillationskolonne bei Drücken von 1,0 bis 2,0 bar und die zweite Destillationskolonne bei Drücken von 1,5 bis 15 bar betreibt.

4. Verfahren nach Anspruch 1 bis 3, dadurch gekennzeichnet, daß die erste Destillationskolonne mit einem Rücklaufverhältnis von 2,0 bis 0,4 betrieben wird.

5. Verfahren nach Anspruch 1 bis 4, dadurch gekennzeichnet, daß der Druck in der zweiten Destillationskolonne so gewählt wird, daß nach vollständiger Kondensation des Kopfproduktes dieser Kolonne dieses bei Temperaturen von mehr als 0 °C als flüssige Phase erhalten wird.

6. Verfahren nach Anspruch 1 bis 5, dadurch gekennzeichnet, daß man in die Auftrennung eine Vinyloxiran enthaltende Lösung einsetzt, die weniger als 3 Gew.% Wasser enthält.

7. Verfahren nach Anspruch 1 bis 6, dadurch gekennzeichnet, daß die zur Auftrennung gelangende, Vinyloxiran enthaltende Lösung vor Einsatz in die erste Destillationskolonne mit einem Stabilisator oder der Lösung eines Stabilisators versetzt wird.

8. Verfahren nach Anspruch 1 bis 7, dadurch gekennzeichnet, daß der Stabilisator dem Kopfprodukt der ersten Destillationskolonne vor Eintritt in die zweite Destillationseinheit zugesetzt wir.

## Claims

1. Process for the separation, by distillation, of a solution essentially containing vinyloxirane, butadiene, a carboxylic acid containing 1-5 carbon atoms and benzene, characterised in that the solution is fed to a first distillation column and butadiene, vinyloxirane and an amount of the benzene contained in the solution such that 20 to 70 % by weight of benzene are contained in the top product of this first distillation are distilled off via the head under a pressure of 0.5 to 2.5 bars, the carboxylic acid and the rest of the benzene being obtained as the bottom product, and in that the top product of the first distillation is fed to a second distillation column in which butadiene and, if appropriate, small proportions of components having lower boiling points than vinyloxirane are distilled off via the head and a bottom product essentially consisting of vinyloxirane and benzene is obtained, some of which is recycled as a reflux into the first distillation column.

2. Process according to Claim 1, characterised in that a top product which contains 25 to 70 % by weight of vinyloxirane is removed in the first distillation column.

3. Process according to Claim 1 and 2, characterised in that the first distillation column is operated under pressures of 1.0 to 2.0 bars and the second distillation column is operated under pressures of 1.5 to 15 bars.

4. Process according to Claim 1 to 3, characterised in that the first distillation column is operated with a reflux ratio of 2.0 to 0.4.

5. Process according to Claim 1 to 4, characterised in that the pressure in the second distillation column is chosen so that after complete condensation of the top product of this column, this product is obtained as a liquid phase at temperatures of more than 0 °C.

6. Process according to Claim 1 to 5, characterised in that a vinyloxirane-containing solution which contains less than 3 % by weight of water is employed in the separation.

7. Process according to Claim 1 to 6, characterised in that a stabiliser or a solution of a stabiliser is added to the vinyloxirane-containing solution used in the separation before the solution is fed into the first distillation column.

8. Process according to Claim 1 to 7, characterised in that the stabiliser is added to the top product of the first distillation column before entry into the second distillation unit.

## Revendications

1. Procédé pour séparer les composants d'une solution contenant principalement du vinyloxirane, du butadiène, un acide carboxylique présentant 1 à 5 atomes de carbone et du benzène par distillation, caractérisé en ce qu'on fait arriver la solution à une première colonne de distillation et on sépare par distillation en courant aérien à une pression de 0,5 à 2,5 bars le butadiène, le vinyloxirane et autant du benzène contenu dans la solution qu'il en faut pour que le produit de tête de cette première distillation contienne 20 à 70 % en poids de benzène, l'acide carboxylique et le benzène résiduel étant obtenus comme produit de queue, et en ce qu'on introduit le produit de tête de la première distillation dans une seconde colonne de distillation dans laquelle le butadiène et, le cas échéant, de faibles proportions de composants de plus bas point d'ébullition que le vinyloxirane sont séparés par distillation en tête et on obtient un produit de queue principalement formé de vinyloxirane et de benzène qui est en partie recyclé comme reflux dans la première colonne de distillation.

2. Procédé suivant la revendication 1, caractérisé en ce qu'on prélève dans la première colonne de distillation un produit de tête qui contient 25 à 70 % en poids de vinyloxirane.

3. Procédé suivant les revendications 1 et 2, caractérisé en ce qu'on fait fonctionner la première colonne de distillation à des pressions de 1,0 à 2,0 bars et la seconde colonne de distillation à des pressions de 1,5 à 15 bars.

4. Procédé suivant les revendications 1 à 3, caractérisé en ce qu'on fait fonctionner la première colonne de distillation avec un rapport de reflux de 2,0 à 0,4.

5. Procédé suivant les revendications 1 à 4, caractérisé en ce qu'on choisit la pression dans la seconde colonne de distillation de manière qu'après la condensation totale du produit de tête de cette colonne, ce produit soit obtenu à des températures de plus de 0 °C comme phase liquide.

6. Procédé suivant les revendications 1 à 5, caractérisé en ce qu'on utilise dans la séparation une solution contenant du vinyloxirane qui renferme moins de 3 % en poids d'eau.

7. Procédé suivant les revendications 1 à 6, caractérisé en ce que la solution contenant du vinyloxirane, devant être séparée en ses composants, est additionnée, avant son introduction dans la première colonne de distillation, d'un agent stabilisant ou de la solution d'un agent stabilisant.

8. Procédé suivant les revendications 1 à 7, caractérisé en ce que l'agent stabilisant est ajouté au produit de tête de la première colonne de distillation avant l'introduction dans la seconde unité de distillation.

FIG. 1